(19) Europäisches Patentamt
European Patent Office
Office européen des brevets

(11) Publication number: **0 273 575 B1**

(12) **EUROPEAN PATENT SPECIFICATION**

(45) Date of publication of patent specification: **15.04.92**

(51) Int. Cl.5: **C07B 35/02**, C07C 9/18, C07C 13/48, C07C 5/03, C07C 5/10, B01J 23/76, B01J 31/02, C10G 49/02, C01B 6/24

(21) Application number: **87310299.0**

(22) Date of filing: **20.11.87**

(54) **Process for the hydrogenation of unsaturated organic compounds using solid catalysts.**

(30) Priority: **21.11.86 FR 8616279**

(43) Date of publication of application:
**06.07.88 Bulletin 88/27**

(45) Publication of the grant of the patent:
**15.04.92 Bulletin 92/16**

(84) Designated Contracting States:
**BE DE GB IT NL**

(56) References cited:
**EP-A- 0 208 405**
**US-A- 3 883 607**

**CHEMICAL ABSTRACTS, vol. 102, no. 8, 25th February 1985, pages 382-383, abstract no. 68018s, Columbus, Ohio, US; M.A. ULLA et al.: "Correlation of solid-phase properties with the hydrogenating activity of lanthanum nickelate (LaNi03) and lanthanum cobaltate (LaCo03)", & ACTAS SIMP. IBEROAM. CATAL. 9TH 1984, 1, 721-30**

(73) Proprietor: **The British Petroleum Company p.l.c.**
**Britannic House Moor Lane**
**London EC2Y 9BU(GB)**

(72) Inventor: **Bonnelle, Jean-Pierre**
**31/1 Avenue Gustave Delory**
**F-59100 Roubaix(FR)**
Inventor: **Pinabiau, Maryse**
**BP France 10 Ouai Paul Doumer**
**F-02412 Courbevoie Cedex(FR)**
Inventor: **Marcq, Jean Phillippe**
**BP France 10 Ouai Paul Doumer**
**F-02412 Courbevoie Cedex(FR)**

(74) Representative: **Richardson, Derek et al**
**BP INTERNATIONAL LIMITED Patents & Agreements Division Chertsey Road**
**Sunbury-on-Thames Middlesex TW16 7LN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

CHEMICAL ABSTRACTS, vol. 100, no. 11, 12th March 1984, page 491, abstract no. 85046a, Columbus, Ohio, US; M. CRESPIN et al.: "Ethylene hydrogenation over reduced lanthanum nickelate (LaNi03) perovskite", & NOUV. J. CHIM. 1983, 7(8-9), 477-81

## Description

The present invention relates to a process for the hydrogenation of unsaturated organic compounds using solid hydrogenation catalysts.

Solid catalysts, for example nickel-containing catalysts, are known for the hydrogenation of unsaturated organic compounds.

Our copending European application publication No. 0208405 published on 14th January, 1987, i.e. after the priority date of 21st November, 1986, claimed for the present application, on an application having a filing date before the aforesaid priority date describes and claims rare earth oxyhydrides of the formula:-

$RE_{1-q}G_qNi_{5-p}M_pO_yH_x$

wherein RE is a rare earth element or a mixture of rare earth elements,
G is either Th and/or Y,
M is at least one of Cu, Mn, Al, Fe, Cr or Co,
q is zero or a number less than 1,
p is zero or a number less than 5,
y is an integer or decimal number from 0.4 to 6.5, and
x is an integer or a decimal number equal to or less than 15, and their preparation.
Whilst no uses for the rare earth oxyhydrides in general are disclosed, there is a passing mention in Example 5 that an oxyhydride having the stoichiometry of $MmNi_5O_{1.7-w}$ $(WO_3)_{2w}H_8$ and a specific surface area of approximately 20 $m^2/g$ proved very active in the hydrogenation of isoprene.

We have now found that rare earth oxyhydrides are effective as catalysts for the hydrogenation of unsaturated compounds.

Accordingly, the present invention provides a process for the hydrogenation of an unsaturated organic compound by contacting the unsaturated organic compound with a solid hydrogenation catalyst under hydrogenation conditions characterised in that the solid hydrogenation catalyst comprises a rare earth oxyhydride having the formula: $Tr_{1-q}G_qNi_{5-p}M_pO_yH_x$ (I) wherein

- Tr is a rare earth element, G is either Th and/or Y,
- M is a metal selected from the group consisting of Cu, Mn, Al, Fe, Cr, Co, Rh, Ru, Re, Pt, Pd, Ir, Os, Mo, V, Ti, W and mixtures of two or more thereof,
- q is zero or a number less than or equal to 1,
- p is zero or a number less than or equal to 5,
- y is a whole number or decimal number in the range from 0.4 to 6.5, and
- x is zero or a whole number or decimal number equal to or less than 15 and with the proviso that the process is other than the hydrogenation of isoprene in the presence of an oxyhydride having the stoichiometry $MmNi_5O_{1.7-w}(NO_3)_{2w}H_8$ and a specific surface area of approximately 20 $m^2/g$.

Rare earth oxyhydrides are sometimes referred to as intermetallic rare earth compound (IREC) oxyhydrides.

By rare earth element is meant elements ranging from lanthanum (atomic number 57) to lutetium (atomic number 71), as well as their mixtures. Among the mixtures, the mixture called Mischmetal (Mm) is particularly recommended, this being a mixture of rare earths having a composition which varies according to the supplier. A Mischmetal is commercially available in the following composition (% by weight): La 33%, Ce 48%, Pr 13%, Nd 4% and other rare earths 2%. A feature of the aforesaid composition is the high cerium (Ce) content.

A suitable cerium-free Mischmetal has the following composition: La 63%, Pr 25%, Nd 8%, other rare earths 4%.

A suitable Mischmetal-Nickel alloy has the following composition: Ni 68.6%, Fe 0.16%, $O_2$ 0.05%, Mm 31.3%.

A suitable cerium-free Mischmetal-Nickel alloy ($MmCe_oNi_5$) has the following composition:- Ni 68.2%, Fe 0.21%, $O_2$ 0.45% and Mm 31.4%

A suitable naturally occuring form of Mischmetal is the mineral Bastnasite which has the composition as follows: Ce 48 - 50%, La 32 = 34%, Pr 13 - 14%, Nd 4 - 5%, other rare earths 2%.

An advantageous composition of Mischmetal comprises the following constituents: Ce 47 - 50%, La 23 - 34%, Pr 5 - 14%, Nd 4 - 18%, other rare earths 2 - 4%.

In all the foregoing compositions, the percentages are expressed by weight.

In the formula (I), M is preferably a metal selected from the group consisting of Cu, Mn, Al, Fe, Cr, Co, Rh and Ru.

In the formula (I), y is preferably a whole number or decimal number in the range from 1 to 3.5 and x is preferably a whole number or decimal number in the range from 1 to 8.

The preferred rare earth oxyhydrides include the oxyhydrides having the following formulae:

$MmNi_5O_yH_x$ (II)
$CeNi_5O_yH_x$ (III)
$LaNi_5O_yH_x$ (IV)
$PrNi_5O_yH_x$ (V)

Among the rare earth oxyhydrides free from cerium, the following compound must be mentioned:

$MmCe_oNi_5O_yH_x$ (VI)

Among the other interesting rare earth oxyhydrides, the following may be mentioned:

$CeNi_{5-k}M_kO_yH_x$ (VII)
$Ce_{1-s}Ni_5G_sO_yH_x$ (VIII)
$MmNi_{5-k}M_kO_yH_x$ (IX)
$Mm_{1-5}Ni_5G_sO_yH_x$ (X)

In the formulae (II) to (X), Mm is Mischmetal, x and y have the same meaning as in formula (I); k is 0 or a whole number or decimal less than 5; s is 0 or a number less than 1.

The rare earth oxyhydrides may be supported on a suitable support. Suitable supports include alumina, silica-alumina, carbon, and the like.

The rare earth oxyhydrides of formula (I) useful in the process of the present invention may be prepared from rare earth metals or rare earth metal alloys in a variety of different ways, generally by processes which involve a number of steps.

A first process, denoted hereinafter as process (A), comprises the steps as follows:

in a first step subjecting an IREC of the formula:

$Tr_{1-g}G_gNi_{5-p}M_p$ (XI)

in a closed reactor, preferably an autoclave, to a plurality of hydriding/dehydriding cycles thereby to form a hydride, the hydriding being performed under a hydrogen pressure of from 20 to 200, preferably from 50 to 100 bar, and at a temperature of from 20 to 450 °C, preferably about 350 °C, and the dehydriding being performed in the same closed reactor at a temperature in the range from 20 to 450 °C,

in a second step oxidising the hydrided product obtained in the first step, and

in a third step chemically reducing the product obtained in the second step.

In the first step of the process (A) the hydrogen pressure may be maintained by compensating for the quantity of hydrogen absorbed by the compound of formula (XI). To effect dehydriding, the closed reactor may be placed under subatmospheric pressure and re-heated to the temperature at which hydriding was performed, i.e. a temperature in the range from 20 to 450 °C. Following the second dehydriding cycle hydrogen fixing will generally be found to be easier and the pressure may thereafter be reduced. The hydriding/dehydriding may suitably be performed on the IREC in lump or block form, which forms subsequently disintegrate. The specific surface area of the powder thereby formed is observed to increase with the number of hydriding/dehydriding cycles to which it is subjected. Suitably from 4 to 20 cycles may be carried out.

In the second step the hydride is obtained in the first step is oxidised, suitably by contact with an oxygen-containing gas or gaseous oxidant, for example air or oxygen, at a temperature in the range from 20 to below 450 °C. This may be achieved either by allowing the hydride obtained in the first step to cool in contact with the oxygen-containing gas or gaseous oxidant, the temperature of which is lower than that of the hydride, or by preheating the aforesaid gas to a temperature below that of the hydride before contact therewith. The oxidation may suitably be carried out by heating the hydride in a current of $O_2/N_2$ for about 15 hours at about 400 °C. Generally, the amount of oxygen which can be fixed increases with an increase in the oxidation temperature.

Thereafter, the product from the second step is chemically reduced in the third step to produce the rare earth oxyhydride. This may suitably be effected by contacting the product at elevated temperature, preferably at about 300 °C, with hydrogen, either under batch or continuous flow conditions.

A second process for the production of rare earth oxyhydrides, denoted hereinafter as process (B), comprises the steps as follows:

in a first step forming a solution comprising soluble salts of nickel and the other metallic element(s) thermally decomposable to the oxides thereof in such proportions as to satisfy the stoichiometric relationship of formula (I), and thereafter precipitating a mixture of the thermally decomposable salts of nickel and the other element(s),

in a second step partially or wholly thermally decomposing the mixture of heat decomposable salts obtained in the first step, and

in a third step reducing the product obtained in the second step in the presence of hydrogen.

As regards the first step, the solution may suitably be formed by dissolving the salts of the metals thermally decomposable to their oxides in appropriate proportions in a suitable solvent, for example water. A preferred method of forming the solution, however, comprises reacting the rare earth intermetallic compound with a suitable acid. In this manner the solution so-obtained has the stoichiometry dictated by the formula (I). A preferred salt thermally decomposable to the oxide is the nitrate, though other salts may be employed. Thus it is preferred, in the preferred method of performing the first step to react the IREC with aqueous nitric acid.

Precipitation of a mixture of the thermally decomposable salts may be achieved in a variety of ways. A suitable method is to evaporate the solution until the salts precipitate out. Alternatively, precipitation may be effected by the addition of a suitable reagent. Suitably precipitation may be accomplished by the addition of a basic solution or other precipitant drop by drop to the solution of salts, preferably the nitrates. As the basic solution there may suitably be used an amine, for example triethylamine, or a carbonate. It is preferred, thereafter, to dry the precipitate so-obtained. This step may suitably be effected in the presence of a support, for example alumina, silica-alumina, carbon, or the like. In this way it has been found possible to produce, for example, $LaNi_5O_{1.7}H_{10}$ supported on alumina.

In the second step the mixture of heat decomposable salts is partially or wholly decomposed. Partial decomposition may suitably be achieved in the case of nitrate salts, for example, by calcination at a temperature in the range from 400 to 800 °C for a period sufficient to achieve partial decomposition, e.g. 15 hours. Total decomposition may suitably be achieved by calcination at 800 °C for a period sufficient to achieve total decomposition, e.g. 15 hours. At a calcination temperature of 800 °C, there is no nitrate residue, but a reduction in the specific surface area is observed. Calcination is preferably carried out at a temperature above 450 °C and below 800 °C, more preferably from 500 to 600 °C.

In the third step the product obtained in the second step is reduced in the presence of hydrogen under appropriate conditions, for example at 300 °C under atmospheric pressure.

The oxyhydride obtained by calcination at 400 °C (i.e. still containing some nitrates) and subsequent reduction at 300 °C under hydrogen at atmospheric pressure conforms to the stoichiometry:

$MmNi_5O_{1.7-w}(NO_3)_{2w}H_x$ (XIII)

wherein w is less than 0.1.

This stoichiometry was calculated by mass balance from thermogravimetric data. This oxyhydride is somewhat unique in that it has a specific surface area of approximately $20m^2/g$, whilst the oxyhydrides prepared by other methods generally have surface areas of less than $10m^2/g$.

A preferred process for the production of rare earth oxyhydrides, denoted hereinafter as process (C) is a modification of process (B). The modification concerns the first step of process (B) wherein, instead of adding a basic solution to the solution of the salts in order to effect precipitation, the solution of salts is added portionwise to an excess of the base under conditions of efficient mixing, thereby to effect precipitation at constant pH. In all other respects process (C) is identical to process (B). An advantage of process (C) is that the rare earth oxyhydrides so-produced have very high surface areas, for example 100 to 120 $m^2/g$.

It can be seen that the processes (A) to (C) enable a product to be obtained in the form of a powder in the first step, the oxidised product, hereinafter to be referred to as the precursor oxide, to be obtained in the second step and the oxyhydride to be obtained in the third step.

It will be observed that the amount of hydrogen fixed in the third step increases with the degree of oxidation in the second step. The more oxygen the precursor oxide contains (the higher the content), the more hydrogen it will fix (x increases).

During the third step (hydrogenation and formation of the oxyhydride) there is a simultaneous reduction and incorporation of hydrogen atoms.

Starting with the precursor oxide of a Mischmetal-Nickely alloy, the following reaction is observable at

300 ° C and at a hydrogen pressure of 1 bar

$$MmNi_5O_{6.7} + 9H_2 \rightarrow MmNi_5O_{1.7}H_8 + 5H_2O \qquad (XIV)$$

(XIV)

This reaction, which leads to a reduction in the proportion of oxygen and the inclusion of hydrogen is very different from the standard hydrogenation, since the "hydriding" proportion is irreversible.

The hydrogen incorporated in the oxyhydride of formula (XIV), may be used as a reductant when the latter is contacted with compounds capable of being hydrogenated, i.e. unsaturated organic compounds.

By contacting the oxyhydride of formula (XIV) with an olefin for example at a temperature of 150 ° C the following reaction takes place:-

$$MmNi_5O_{1.7}H_8 + 4\ (Olefin) \rightarrow MmNi_5O_{1.7} + (alkane)$$

(XIV) (XV)

By contacting the compound of formula (XV) with hydrogen at atmospheric pressure and at ambient temperature the original oxyhydride of formula (XIV) can be re-formed according to the equation:

$$MmNi_5O_{1.7} + 4H_2 \rightarrow MmNi_5O_{1.7}H_8$$

The foregoing reactions form the basis for using rare earth oxyhydrides as hydrogenation catalysts. In fact, the rare earth oxyhydride is able to donate its hydrogen to a reducible compound, replenish itself with hydrogen, again donate its hydrogen, again replenish itself and so on.

The catalytic activity of the oxyhydride depends on a number of factors including the values of x and y in the formula (I) and its specific surface are. The specific surface area in turn depends upon the process used for its preparation. The precursor oxides possess a specific surface area of the order of 10 $m^2/g$ when prepared by process (A) and of the order of 20 $m^2/g$ when prepared by process (B), provided that the calcination temperature is below 600 ° C. In passing from the precursor oxide form to the oxyhydride form the specific surface area increases considerably, and may attain a value of up to 50 $m^2/g$. As mentioned hereinbefore, it is an advantage of process (C) that it can result in specific surface areas of the order of 100 to 120 $m^2/g$.

The unsaturated organic compound may suitably be an olefinically unsaturated compound or an acetylenically unsaturated compound, preferably an olefinically unsaturated compound. The olefinically unsaturated compound may be monoolefinically unsaturated, diolefinically unsaturated, polyolefinically unsaturated or aromatically unsaturated. The unsaturated compound may be an aliphatic, cycloaliphatic or aromtic compound, or a combination thereof. Examples of unsaturated compounds which may be hydrogenated in the process of the present invention include olefins, for example alkenes, cycloalkenes and aralkenes, monocyclic aromatic hydrocarbons, for example benzene and alkylbenzenes, polycyclic aromatic hydrocarbons, for example naphthalene, heterocyclic aromatic hydrocarbons containing nitrogen in the aromatic ring, for example pyridine, or containing sulphur in the aromatic ring, for example thiophene, or a mixture of two or more thereof.

The nature of the hydrogenation conditions will depend on the nature of the unsaturated compound to be hydrogenated. These conditions are well established in the art and are further illustrated in the following Examples.

It is believed that the hydrogen contained in the oxyhydrides is present either as surface hydrogen, which is immediately available for reaction, or incorporated in the body of the oxyhydride. On contact with unsaturated compounds the oxyhydride it is believed donates the surface hydrogen, which is then replaced by hydrogen from the body. On the other hand, in the presence of added gaseous hydrogen it is believed that everything occurs as though the hydrogen on the surface of the oxyhydride is replaced by the hydrogen deriving from the body of the oxyhydride, which is itself replaced by the external hydrogen. Whilst we do not in any way wish to be held to these beliefs, it is nevertheless possible to operate the process in the absence of added gaseous hydrogen, but the reduction reaction rate decreases with time. However, it is preferred to operate the process in the presence of additional hydrogen.

It is an advantage of the use of rare earth oxyhydrides in the process of the invention that in

comparison with many prior art hydrogenation catalysts, for example metals and metal oxides, they are appreciably more tolerant to sulphur, or expressed in another way they are appreciably more resistant to sulphur poisoning. Moreover, it has been established that using feeds containing for example pyridine and/or naphthalene the hydrogenation activity relative to naphthalene and the hydrogenolysis activity relative to pyridine is largely independent of the hydrogen pressure. This is important because taken together with the finding that the rare earth oxyhydrides are substantially insensitive to dimethyl disulphide (DMDS) and thiophenes it indicates that it may be possible to hydrogenate naphthalene, pyridine, DMDS, thiophene and other aromatic products during different hydrotreatments of mineral oil-derived feedstocks, for example hydrocracking, hydrodesulphurisation, hydrodenitrification, hydroisomerisation and hydrogenation at low hydrogen pressures. This is to be compared with catalysts currently in use for which it is necessary to operate at high pressures.

The process of the present invention will now be further illustrated by the following Examples.

Example A

Preparation of an Oxyhydride from a Hydride [Illustrative of preparative process (A)]

The hydride was produced from $MmNi_5$. The hydrogenation was carried out in five cycles at 450 °C, at a hydrogen pressure of 150 bars.

The compound with the formula $MmNi_5H_6$ was thus obtained.

This hydride was then oxidised with air (in a muffle furnace with an $O_2/N_2$ current) for 15 hours at 450 °C, to give the compound with the formula $MmNiO_{6.76}$.

This precursor oxide was reduced in a current of $H_2$, at atmospheric pressure and at 300 °C.

The oxyhydride with the formula $MmNi_5O_{0.76}H_8$ was then obtained.

Examples B and C

Preparation of an Oxyhydride from Nitrates of Nickel and Rare Earths Illustrative of Preparative Process (B)

The first stage involved the preparation of a solution of nickel nitrate and rare earth nitrates.

- either from Ni and Mm, in a stoichiometric ratio of Ni:Mm = 5;
- or by attacking the metal alloy $NmNi_5$ with $HNO_3$ which enables a satisfactory stoichiometric ratio to be obtained directly.

The solution was then evaporated at approximately 80 °C until the nitrates precipitate. The precipitate was then dried.

In the second stage the precipitate was decomposed by eliminating $NO_2$. The black residue was then calcined.

In carrying out Example B calcination was effected at 400 °C, which did not allow the nitrates to be completely eliminated.

In the course of Example C the precipitate was calcined at 800 °C for 15 hours (which completely eliminated the nitrate but reduced the specific area of the intermediate precursor oxide and that of the oxyhydride).

In both cases the hydrogenation was effected at 300 °C at atmospheric pressure.

According to Example B (calcination at 400 °C) the oxyhydride with the formula: $MmNi_5O_{1.7-w}(NO_3)_{2w}H_8$ was obtained,
where w denotes a value less than 0.1,
and according to Example C (calcination at 800 °C), the oxyhydride with the formula: $MmNi_5O_{1.7}H_8$ was obtained.

The oxyhydride prepared by process (B) exhibits a specific area approximately twice that of the oxyhydride prepared in Example A from the hydride, provided that too high a temperature (exceeding 700 °C) is avoided during calcination.

The specific areas measured on the precursor oxides (before reduction by hydrogen) were approximately 20 $m^2/g$ for the oxide prepared in Example B, and calcined at 400 °C, and approximately 5 $m^2/g$ for the oxide prepared in Example C and calcined at 800 °C, both obtained from nitrates, and approximately 10 $m^2/g$ for the oxide in Example A obtained from the hydride.

It was not possible to measure the specific area of the oxyhydrides obtained after the third stage. It appears, however, that these specific areas were increased considerably during the third stage.

Example 4 - (Illustrative of Preparative Process (C))

The method comprised five different steps:-

1. Precipitation
2. Filtration
3. Drying
4. Oxidation
5. Sieving

Step (I) - Precipitation

(a) Materials

Peristaltic pump: Masterflex 7016 (Bioblock scientific)
Electric stirrer with glass screw
Teflon tubing
Milli-Q-grade water (Millipore)
$Ce(NO_3)_3.6H_2O$ (cerium(III)nitrate), rectapurgrade (Prolabo)
$Ni(NO_3)_2.6H_2O$ (nickel(II)nitrate), normapurgrade (Prolabo)
Triethylamine (for synthesis) (Prolabo) (TEA)

(b) Procedure

Cerium and nickel nitrates were weighed and dissolved in water until the total concentration of nitrates salts was about 275 g/litre.

The nitrates solution was dripped continuously (2.5 ml/minute) into a large excess of continuously stirred TEA (4 litre of TEA minimum per litre of nitrates solution). The stirrer speed was set to give rapid agitation without causing a vortex.

Each nitrate solution drop dripping into the TEA reacted quickly and hydroxides precipitated. The precipitate was ejected from the reaction zone and concentrated on the wall of the flask. TEA is not soluble in water. Since the density of TEA is lower than that of water, the upper part of the liquid phase in the vessel remained nearly pure TEA. In accordance the reaction zone contained nearly pure TEA throughout and the drops kept precipitating instantaneously.

Step (2) - Filtration

Two different methods were used:

(a) filtration on a glass filter and washing with methanol in order to eliminate the remaining TEA and subsequent washing with water in order to remove the remaining methanol, and
(b) centrifugation at 3000 r.p.m. and washing with methanol repeated twice and thereafter washing the precipitate with fresh water.

(3/4) Drying and Oxidation (Calcination)

The washed powder from step (2) was first dried at 100°C for 15 hours and thereafter calcined in a quartz reactor at 430°C using an air flow of 40 ml/minute for 24 hours.

(5) Sieving

For the pilot plant and microreactor experiments only the fraction having particles sized between 37 and 250 microns were selected.

EXAMPLES OF CATALYTIC USAGE

Examples 1 - 13

These Examples, which are summarised in Table 1 below, relate to the hydrogenation of isoprene at 20°C with $H_2$ at atmospheric pressure, using oxyhydrides as catalysts. The Examples differ in the initial

material, the preparative process, the oxidation temperature and in the specific are at the precursor oxide stage.

In Table 1 the columns contain the following information.

Column 1 indicates the number of the Example;

Column 2 indicates the general formula of the oxyhydride used;

Column 3 indicates the preparation process used (A) or (B), the product obtained in step I, which is used as the initial (raw) material for step II (oxidation);

Column 4 indicates the oxidation temperature (° C) and the specific area ($m^2/g$) of the precursor oxide;

Column 5 indicates the values of y and x, i.e. the molar contents of oxygen and hydrogen;

Column 6 indicates the activity of the oxyhydride, expressed in $mmols/h/m^2$ (mmols of isoprene obtained per hour and per $m^2$ of specific area of the catalyst, measured in the precursor oxide state);

and the selectivity, expressed in terms of the result of the hydrogenation of isoprene

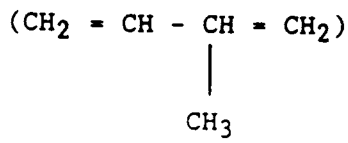

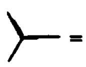 corresponds to 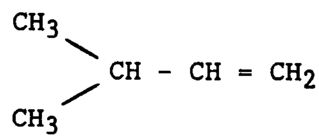

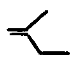 corresponds to 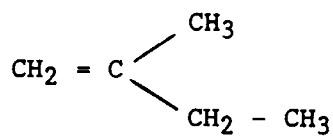

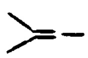 corresponds to 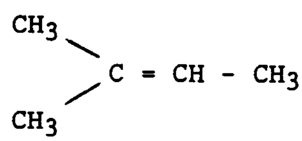

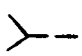 corresponds to 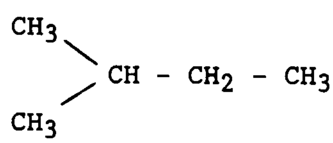

It will be seen that the first three of these compounds are isopentenes representing an intermediate reduction stage, whilst the last compound is the isopentane representing the final reduction stage.

INTERPRETATION OF THE RESULTS

The following observations may be made:

Catalytic activity varies according to the hydrogen (x) and oxygen (y) content of the oxyhydride.

If these values are low (Examples 1, 2, 3, 5), oxhydrides are obtained which behave like hydrides and lead essentially to isopentenes, with practically no isopentane obtained, whereas the compounds where x and y have high values (Examples 6 to 13) give a high proportion of isopentane.

It will also be observed that the oxyhydrides prepared by preparative process (B) exhibit a higher specific area, which also favours the production of isopentane.

Table I also indicates the size of the specific area, which depends on the oxyhydride preparation process, and particularly on the calcination temperature. If the calcination temperature is too high (800° C for Example 7), less isopentane is produced than in Example 6 (calcination temperature 400° C).

Example 14

This Example illustrates the reduction of isopentene by means of an oxyhydride, at a temperature of 150 ° C, in the absence of hydrogen, using helium as a carrier.

This Examples enabled the total hydrogen content and the rate of retrodiffusion of the bulk hydrogen (inside the oxyhydride) to the surface of the oxyhydride to be determined.

TABLE I

| Example No. | Oxyhydride formula | Preparative Process | Product Obtained at Stage I' | Stage II: Oxidation | | Stage III: $H_2$ Reduction/inclusion | | Hydrogenation of isoprene P(H2) = 1 bar ; Temp = 20°C | | | | |
|---|---|---|---|---|---|---|---|---|---|---|---|---|
| | | | | (°C) | ($m^2/g$) (1) | y | x | Activity mmol/h/$m^2$ | Selectivity 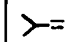 | 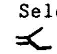 | 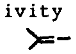 | 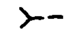 |
| 1 | $LaNi_5Oy$ Hx | A | Hydride | 20 | 1.2 | 0.44 | 0.04 | $2.6.10^{-5}$ | 10 | 100 | 0 | 0 |
| 2 | $LaNi_5Oy$ Hx | A | Hydride | 400 | 1.2 | 1.5 | 0.3 | $8.0.10^{-5}$ | 0 | 0 | 100 | 0 |
| 3 | $MmNi_5$ Oy Hx | A | Hydride | 20 | 0.5 | 0.1 | 0.04 | $7.2.10^{-4}$ | 0 | 24 | 50 | 26 |
| 4 | $MmNi_5$ Oy Hx | A | Hydride | 400 | 1.7 | 1.7 | 8 | $5.0.10^{-2}$ | 0 | 0 | 0 | 100 |
| 5 | $MmCeoNi_5$ OyHx (2) | A | Hydride | 400 | - | 1.7 | 0.4 | $1.5.10^{-3}$ | 18.1 | 35.3 | 43.5 | 3.1 |
| 6 | $MmNi_5$ Oy Hx | A | $MmNi_5$ Dissolved in $HNO_3$ | 400 | 20.3 | 1.7 | 2.1 | $1.0.10^{-3}$ | 0.3 | 0.7 | 1.8 | 97.2 |
| 7 | $MmNi_5$ Oy Hx | B | | 800 | 5.3 | 1.7 | 2.7 | $6.4.10^{-3}$ | 1.4 | 5.5 | 11.6 | 81.5 |
| 8 | $MmNi_5$ Oy Hx | B | Nitrates | 400 | 17.6 | 1.7 | 5.2 | $1.1.10^{-3}$ | 0 | 0 | 0 | 100 |
| 9 | Ce $Ni_5$ Cy Hx) | B | Nitrates | 400 | 18.5 | 2.0 | 4.45 | $1.0.10^{-3}$ | 0.5 | 0.9 | 2 | 96.6 |
| 10 | $LaNi_5Oy$ Hx ) | | | 400 | 21.6 | 1.5 | 3.4 | $1.1.10^{-3}$ | 0 | 0 | 0 | 100 |
| 11 | $LaNi_5$ Oy Hx ) | | | 400 | 8.7 | 1.5 | 4 | $1.1.10^{-3}$ | 0 | 0 | 0 | 100 |
| 12 | $PrNi_5Oy$ Hx ) | | | 400 | 16.7 | 1.8 | 5.15 | $1.0.10^{-3}$ | 0.4 | 1.3 | 2.4 | 95.9 |
| 13 | $NdNi_5$ Oy Hx ) | | | 400 | 11.7 | 1.5 | 4.35 | $1.0.10^{-3}$ | 0.5 | 0.6 | 3.9 | 95.2 |

(1) The activity is expressed in millimols of isoprene reduced per hour and in $m^2$ or specific area in stage II of the preparation.
(2) MmCeo indicates Mischmetal without cerium.

The reaction is as follows:

$MmNi_5O_{1.7}H_8$ + 4 (olefin) → $MmNi_5O_{1.7}$ + 4 (alkane)

This reaction enables the relative rate of hydrogenation to be represented as a function of the relative hydrogen concentration of the oxyhydride.

Figure 1 indicates, on the x-axis, the relative hydrogen concentration of the oxyhydride, and on the y-axis the relative rate of hydrogenation.

This curve may be divided into three zones, zone I corresponding to the surface hydrogen (+/- 15% of the total hydrogen content). During the initial stage of the hydrogenation, corresponding to this zone I, only isopentane is obtained. Total isopentane hydrogenation requires rapid diffusion of the hydrogen, which may be limited by the retrodiffusion of the mass hydrogen to the surface of the oxyhydride. In zone II, only isopentene (mono-olefin) is obtained, which indicates limited retrodiffusion. In zone III (after 1 hour), when the hydrogen content of the oxyhydride is reduced to 25% of the initial content, the hydrogenation continues but it slows down considerably.

Example 15

The isoprene was hydrogenated under the same conditions as in Example 14; except that the intake of isoprene was stopped for approximately 20 minutes after the first hour of reaction.

When the isoprene intake was resumed, the relative speed of hydrogenation was shown to have increased substantially, but this increase in speed only lasted a few minutes, then reversed to its value before the isoprene intake was stopped.

This test shows that there is retrodiffusion of the mass hydrogen to the surface of the oxhydride.

Rare earth oxyhydrides and IREC oxyhydrides may be used advantageously as catalysts for the hydrogenation not only of olefins, but also of other unsaturated compounds, particularly monocyclic aromatic hydrocarbon compounds such as benzene, polycyclic aromatic compounds such as naphthalene, heterocyclic compounds containing nitrogen in their nucleus, such as pyridine, or sulphur in their nucleus, such as thiophene.

Example 16 and Comparative Example 17

Hydrogenation of Naphthalene

Example 16 illustrates the hydrogenation of naphthalene, in a pilot installation, with hydrogen at a pressure of 160 bars, at a temperature of 100 °C, the naphthalene being present in n-heptane at a concentration of 2% (% higher than present in practice).

An oxyhydride with the formula: $MmNi_5O_{1.7}H_8$ is used as the catalyst.

Comparative Example 17 illustrates the same hydrogenation as Example 16, the difference being that the oxyhydride used as a catalyst in Example 16 was replaced by a hydride obtained from $MmNi_5$.

The results are shown in Figure 2, the x-axis representing the time expressed in hours, and the y-axis representing the percentage of reduced naphthalene by weight at a given moment, the reduction taking place instantaneously.

Curve A corresponds to Example 16, using the oxyhydride as the catalyst, and curve B corresponds to Example 17, using the hydride of $MmNi_5$ as the catalyst.

It is seen that the catalytic activity of the oxhydride is much greater than that of the hydride, and also that this activity remains practically constant, even after a period of 1750 hours (72 days), whilst the catalytic activity of the hydride decreases fairly rapidly with the time on stream.

The naphthalene ($C_{10}H_8$) may be partially reduced to tetralin or tetrahydronaphthalene ($C_{10}H_{12}$), total reduction leading to the formation of decalin or decahydronaphthalene ($C_{10}H_{18}$), Example 16, using the IREC oxyhydride as the catalyst, leads to the formation of a mixture of decalin and tetralin, with a higher quantity of decalin, whilst Example 17, using the IREC hydride as the catalyst, gives only tetralin (partial hydrogenation).

Example 18

Example 16 was reproduced, the difference here being that the reaction temperature was 350 °C, and the initial materials also contained, more than 2% by weight of naphthalene, about 0.1% of dimethyl

disulphide (DMDS) by total weight of the mixture.

The reduction of the naphthalene was carried out for 150 hours (six days).

The catalytic activity is expressed in terms of the percentage conversion of naphthalene (% of reduced naphthalene) on the one hand, and by the hydrogenation activity on the other. The hydrogenation activity also takes into account the degree of reduction of the naphthalene, and is expressed by the relation:

Hydrogenation activity = 0.4. (% Tetraline) + (% Decaline)

The results are shown in Figure 3.

The upper curve C indicates the conversion of the naphthalene (% by weight of reduced naphthalene), the lower curve D expresses the hydrogenation activity, as indicated above.

It will be seen that at a temperature of 350°C (curve C, Figure 3), the percentage of reduced naphthalene is almost twice that obtained at 100°C (curve A, Figure 2).

It will also be seen that due to the presence of sulphur, the activity of the catalyst is slightly reduced in the course of the first day, then remains practically stable.

It will also be seen that, unlike the catalysts consisting of metals or metal oxides, which are quickly contaminated by compounds of sulphur, the oxyhydrides, although reducing their activity at the end of the first day, then maintain a relatively high activity, which remains practically stable, despite the presence of the compounds containing sulphur.

Other tests for the reduction of naphthalene containing thiopene (more difficult to use than DMDS) show that the catalytic activity of the oxyhydride is maintained even if the thiophene content attains a value of 1.5% of the naphthalene content.

Example 19 and Comparative Example 20

Example 19 illustrates the reduction of naphthalene (2% by weight in n-heptane) in the presence of 0.1% by weight of DMDS at 350°C, at different hydrogen pressures, using as catalyst the oxyhydride having the formula: $MmNi_5O_{1.7}H_8$, prepared according to process A. The percentages are expressed in relation to the total weight of the mixture n-heptane + naphthalene + DMDS.

Comparative Example 20 illustrates the reduction of the same product, replacing the oxyhydride with a Nickel catalyst on an alumina support, and varying the hydrogen pressure.

The percentage conversion of the naphthalene was determined, and the result shown in Figure 4, where the x-axis indicates the logarithm of the hydrogen pressure, and the y-axis the logarithm of the naphthalene conversion. The straight line E represents the percentage conversion of the naphthalene, using the oxyhydride as the catalyst, at hydrogen pressures varying from 80 to 150 bars, and straight line F represents the percentage conversion of the naphthalene using $Ni/Al_2O_3$ as a catalyst, at hydrogen pressures from 80 to over 200 bars.

It will be seen that straight line E is horizontal, i.e. using oxyhydride as a catalyst the percentage conversion of the naphthalene is independent of the hydrogen pressure, whilst straight line F shows that when nickel on an alumina support is used as the catalyst, the conversion of the naphthalene is a function of the square of the hydrogen pressure.

The two curves intersect at point P, which corresponds to a hydrogen pressure of 180 bar.

In other words, the use of an oxyhydride with the formula $MmNi_5O_{1.7}H_8$ as a catalyst enables hydrogen to be used at reduced pressure, instead of working at a high hydrogen pressure with a $Ni/Al_2O_3$ catalyst.

Examples 21 - 25

These Examples illustrate the hydrogenation of naphthalene and pyridine in the presence and in the absence of sulphur-containing compounds (DMDS).

Use is made of 12.6 g of oxyhydride of the same type as that used in Example 6 for the hydrogenation of isoprene.

This oxyhydride, prepared from $MmNi_5$, dissolved in $HNO_3$ and calcined at 400°C, had a specific area of 20.3 $m^2/g$ in the precursor oxide state.

The conversion of the precursor oxide to oxyhydride was carried out, for convenience of handling, at a hydrogen pressure of 150 bar and at a temperature of 300°C.

The composition to be hydrogenated contained, in n-heptane, 2% naphthalene, p% pyridine and q% DMDS.

The conversion of naphthalene (% of reduced naphthalene) was measured, together with the hydrogenation activity of the naphthalene, expressed by the relation:

Hydrogen activity of the naphthalene = 0.4. (% Tetralin) + (% Decalin).

The conversion of pyridine (% of reduced pyridine) was also measured.

The results are given in Table II, where the columns contain the following information:

Column 1 indicates the number of the Example;

Column 2 indicates the hydrogen pressure in bar;

Column 3 indicates the value of q, i.e. the % by weight of DMDS in the composition to be hydrogenated;

Column 4 indicates the value of p, i.e. the % by weight of pyridine in the composition to be hydrogenated;

Column 5 indicates the conversion of naphthalene in % by weight;

Column 6 indicates the hydrogenation activity of naphthalene in %,

Column 7 indicates the conversion of pyridine in %.

TABLE II

| Example No. | $P(H_2)$ (bar) | q. (%DMDS) | p. (%pyridine) | Conversion of Naphthalene (%) | Hydrogenation activity of Naphthalene (%) | Conv. of Pyridine (%) |
|---|---|---|---|---|---|---|
| 21 | 80 | 0.1 | 0 | 40 | 17 | 0 |
| 22 | 80 | 1 | 0 | 44 | 18 | 0 |
| 23 | 80 | 1 | 1 | 12 | 5 | 58 |
| 24 | 150 | 0.1 | 0 | 41 | 18 | 0 |
| 25 | 150 | 0.1 | 1 | 17 | 7 | 74 |

The following observations may be made:

The pyridine is completely reduced and converted to ammonia and pentane. No trace is found of piperidine, which constitutes the first stage of pyridine reduction.

The naphthalene is hydrogenated in almost the same way in the presence and absence of DMDS, there being no competition between the naphthalene and DMDS during hydrogenation.

This result, obtained with an oxyhydride prepared according to process (B) (precipitation of the nitrates) confirms the result obtained in Example 19, where use is made of an oxyhydride prepared according to process (A) (passing through the hydride stage). The two oxyhydrides, one prepared by process (A) and the other by process (B), prove insensitive to DMDS, and even with a relatively high % of DMDS in the charge, the hydrodesulphurisation is total.

The hydrogenolysing activity of the catalyst relative to the pyridine is very good, but enters into competition with the hydrogenation activity relative to the naphthalene.

Hydrogenolysing activity relative to pyridine is understood to mean the conversion of pyridine to ammonia and pentane.

The hydrogenating and hydrogenolysing action of the oxyhydride catalyst hardly varys with hydrogen pressure. This is important because it indicates that it would be possible to hydrogenate naphthalene, pyridine, DMDS, thiophene and other aromatic products during different hydrotreatments of the mineral-oil charges, such as hydrocracking, hydrodesulphurisation, hydroisomerisation and hydrogenation, at low hydrogen pressures, whilst with the other catalysts currently being used it is necessary to work at high hydrogen pressures.

The use of oxyhydrides as hydrogenation catalysts is therefore advantageous not only from the technical but also from the economic point of view.

Example 26

This Example illustrates the reduction of naphthalene (2% by weight in n-heptane), in the presence of 0.1% by weight of DMDS, at 350 °C, at different hydrogen pressures of between 80 and 150 bar, with a feed rate of 20 g/hour and a hydrogen flow rate of 3 litres/hour, the catalyst weight being 24 g.

This Example differs essentially from Example 19 in the nature of the catalyst used. In fact $MmNi_5O_{1.7}H_8$, prepared by process A (from the corresponding hydride), used in Example 19, was replaced by $CeNi_5O_2H_{4.45}$ prepared from cerium and nickel nitrates in the proportions 1:5, by process (B), by evaporation and precipitation, followed by calcination at 450 °C, then reduction and inclusion of hydrogen at 300 °C.

The results are shown in Table III.

TABLE III

| $H_2$ pressure (bar) | Conversion of Naphthalene (%) | Hydrogenation Activity of Naphthalene (%) |
|---|---|---|
| 80 | 53.2 | 23.5 |
| 100 | 52.6 | 23.3 |
| 125 | 55.9 | 25.8 |
| 150 | 51.8 | 24.4 |

These results confirm those in Example 19, namely that the activity of the catalyst does not vary with hydrogen pressure.

This example also shows that the catalyst used, which is here a synthetic oxyhydride, obtained by preparative process (B), from nitrates of ace and Ni, and purchased commercially, give results similar to those obtained using as a catalyst an oxyhydride prepared by process (A) from the corresponding hydride.

Example 27 (Pilot Plant Hydrogenation)

This Example demonstrates the resistance to sulphur poisoning of the rare earth oxyhydrides, since the catalyst used in this Example is submitted to a very high concentration of $H_2S$.

$CeNi_5O_yH_x$ as produced by preparative process (B) was used as catalyst. Its surface area was 40 $m^2/g$ and its hydrogen content (x) was 4.4.

A feed ($nC_7$ + 2% naphthalene + z% pyridine + w% DMDS was hydrogenated in a pilot installation with hydrogen under the following conditions:-

T (react.) = 350 °C
P ($H_2$) = 80 barg
Reactor vol. = 20 cc
Feed flow = 20 g/h
Hydrogen flow = 2 litres/hour
Catalyst weight = 23.1 g

The results obtained are shown in Table IV.

TABLE IV

| Pyridine/feed (z %)<br>DMDS/feed (w %) | 2<br>0.1 | 2<br>1 | 2<br>2 | 0<br>2 | 0<br>15 | 0<br>2 | 2<br>2 |
|---|---|---|---|---|---|---|---|
| Time on stream (h) | 225 | 97 | 48 | 142 | 3 | 191 | 64 |
| Naphthalene conv. (% $g^{-1}$) | 1.4 | 0.26 | 0.23 | 0.95 | 1.2 | 1.2 | 0.2 |
| Pyridine conv. (% $g^{-1}$) | 0.8 | 1.25 | 1.2 | - | - | - | 1.3 |
| Total conv. (% $g^{-1}$) | 2.2 | 1.51 | 1.43 | 0.95 | 1.2 | 1.2 | 1.5 |

Example 28

This Example demonstrates the effect of hydrogen pressure on the hydrogenation activity of naphthalene, pyridine and quinoline.

$CeNi_5O_yH_x$ produced by preparative procedure (C) was used as catalyst. Its surface area was 72 $m^2/g$ and its hydrogen content (x) was 8.0.

A feed [(i) $nC_7$ + 2% naphthalene + 2% pyridine + 2% DMDS (ii) $nC_7$ + 4% quinoline + 2% DMDS] was hydrogenated in a pilot installation with hydrogen under the following conditions:-

T (react) = 350 °C
P ($H_2$) = 80 - 150 barg
Reactor vol. = 20 cc
Feed flow = 20 g/h
Hydrogen flow = 2 litres/h
Catalyst weight = 18.4 g

The results obtained are shown in Table V.

TABLE V

Feed (i)

| $P (H_2)$ (barg) | 80 | 100 | 150 |
|---|---|---|---|
| Time on stream (h) | 90 | 51 | 42 |
| Naphthalene conv. (% $g^{-1}$) | 0.8 | 0.8 | 0.7 |
| Pyridine conv. (% $g^{-1}$) | 2.1 | 2.1 | 1.7 |
| Total conv. (% $g^{-1}$) | 2.9 | 2.9 | 2.4 |

Feed (ii)

| $P (H_2)$ (barg) | 80 | 100 | 125 | 150 |
|---|---|---|---|---|
| Time on stream (h) | 105 | 62 | 190 | 68 |
| Quinoline hydrogenation: | | | | |
| Total conversion (% $g^{-1}$) | 3.6 | 3.7 | 3.8 | 3.4 |
| Tetrahydroquinol. (% $g^{-1}$) | 3.3 | 3.4 | 3.5 | 3.2 |
| Decahydroquinol. (% $g^{-1}$) | 0.3 | 0.3 | 0.3 | 0.2 |

Example 29

This Example provides a comparison between a commercial catalyst (ex Procatalyse) and two different rare earth oxyhydride catalysts (one obtained by preparative process (B) and the second by preparative process(C)).

The catalysts used were as follows:-

A - Commercial catalyst, ex Procatalyse ($NiPS_2$).

B - $CeNi_5O_yH_x$ wherein x = 4.4 and having a surface area of 40 $m^2$/g prepared by process (B) (nitrates evaporation).

C - $CeNi_5O_yH_x$ wherein x = 8.0 and having a surface area of 72 $m^2$/g prepared by process (C) (TEA precipitation).

A feed ($nC_7$ + 2% naphthalene + 2% pyridine + z% DMDS) was hydrogenated in a pilot plant installation with hydrogen under the following conditions:

| | |
|---|---|
| T (react) | = 350 °C |
| $P (H_2)$ | = 80 barg |
| Reactor vol. | = 20 cc |
| Feed flow | = 20 g/h |
| Hydrogen flow | = 2 litres/h |
| Catalyst weight | = ± 20 g |

The results obtained are shown in Table VI.

TABLE VI

| Catalyst | A | B | C |
|---|---|---|---|
| 0.1% DMDS (feed) | | | |
| Time on stream (h) | 0 -- 1 | 0 -- 1 | 0 -- 1 |
| Naphthalene conv. (% $g^{-1}$) | 0.7 | 1.7 | 3.9 |
| Pyridine conv. (% $g^{-1}$) | 0.06 | 1.0 | 2.8 |
| Time on stream (h) | 130 | 225 | 160 |
| Naphthalene conv. (% $g^{-1}$) | 0.4 | 1.4 | 1.8 |
| Pyridine conv. (% $g^{-1}$) | 0.02 | 0.8 | 2.4 |
| 1% DMDS/Feed | | | |
| Time on stream (h) | - | 97 | 70 |
| Naphthalene conv. (% $g^{-1}$) | 0 | 0.3 | 1.0 |
| Pyridine conv. (% $g^{-1}$) | 0 | 1.3 | 2.3 |
| 2% DMDS/Feed | | | |
| Time on stream (h) | - | 48 | 90 |
| Naphthalene conv. (% $g^{-1}$) | 0 | 0.2 | 0.8 |
| Pyridine conv. (% $g^{-1}$) | 0 | 1.2 | 2.1 |

Example 30

This Example demonstrates the hydrotreatment of a vacuum distillate (D cut Arab medium) in a batch reactor.

The catalyst used was $CeNi_5OyH_x$.

A feed consisting of a D cut Arab medium vacuum distillate was hydrogenated in a batch reactor under the conditions shown in Table VI.

TABLE VI

| | | | |
|---|---|---|---|
| Catalyst ($CeNi_5O_yH_x$) wt. (g) | | 12.7 | 12.6 |
| Feed (D cut AM vac. Distillate) wt (g) | | 20.4 | 20.4 |
| Reaction Conditions | | | |
| Time (hours) | | 2 | 2 |
| Temperature (°C) | | 350 | 350 |
| $P(H_2)$ (barg) | | 10 | 20 |
| | Initial | | |
| Viscosity at 100°C (cSt) | 12.0 | 10.6 | 10.2 |
| Density at 70°C ($gml^{-1}$) | 0.8787 | 0.8713 | 0.8670 |
| Sulphur (%) | 2.4 | 1.4 | 1.1 |
| Nitrogen (%) | 370 | 285 | 280 |
| Aromatics Distribution | Initial | | |
| HPCL data (u.a.) | | | |
| Monoaromatics | 1.2 | 1.3 | 1.7 |
| Diaromatics | 13.5 | 10.3 | 7.4 |
| Fluorenes | 12.8 | 10.0 | 9.6 |
| Anthracenes | 15.2 | 10.4 | 10.1 |
| Pyrenes | 5.4 | 3.3 | 3.0 |
| Chrysenes and above | 7.9 | 5.0 | 3.8 |
| TOTAL | 56.0 | 40.3 | 35.6 |

## Claims

1. A process for the hydrogenation of an unsaturated organic compound by contacting the compound with a solid hydrogenation catalyst under hydrogenation conditions characterised in that the solid hydrogenation catalyst comprises a rare earth oxyhydride having the formula: $Tr_{1-q}G_qNi_{5-p}M_pO_yH_x$ (I) wherein Tr is a rare earth element, G is either Th and/or Y,
   M is a metal selected from the group consisting of Cu, Mn, Al, Fe, Cr, Co, Rh, Ru, Re, Pt, Pd, Ir, Os, Mo, V, Ti, W and mixtures of two or more thereof,
   q is zero or a number less than or equal to 1,
   p is zero or a number less than or equal to 5,
   y is a whole number or decimal number in the range from 0.4 to 6.5, and
   x is zero or a whole number or decimal number equal to or less than 15 and with the proviso that the process is other than the hydrogenation of isoprene in the presence of an oxyhydride having the stoichiometry $MmNi_5O_{1.7-w}(NO_3)_{2w}H_8$ and a specific surface area of approximately 20 $m^2/g$.

2. A process according to claim 1 wherein the unsaturated organic compound is an olefinically unsaturated compound.

3. A process according to either claim 1 or claim 2 wherein the unsaturated compound is selected from olefins, monocyclic aromatic hydrocarbons, polycyclic aromatic hydrocarbons, heterocyclic aromatic hydrocarbons containing nitrogen in the aromatic ring and heterocyclic aromatic hydrocarbons containing sulphur in the aromatic ring, or a mixture of two or more thereof.

4. A process according to claim 1 wherein the unsaturated organic compound is isoprene.

**5.** A process according to claim 1 wherein the unsaturated organic compound is naphthalene.

**6.** A process according to claim 1 wherein the unsaturated organic compound is pyridine.

**7.** A process according to claim 1 wherein the unsaturated organic compound is thiophene.

**8.** A process according to claim 1 wherein the hydrogenation process is a hydrotreatment of a mineral oil-derived feedstock.

**9.** A process according to claim 8 wherein the hydrotreatment is either hydrocracking, hydrodesulphurisation, hydrodenitrification, hydroisomerisation or hydrogenation.

**10.** A process according to any one of the preceding claims wherein the process is operated in the presence of additional hydrogen.

**11.** A process according to any one of the previous claims wherein there is used a catalyst of the formula (I) wherein Tr is Mischmetal.

**12.** A process according to any one of claims 1 to 10 wherein there is used a catalyst of the formula (I) wherein Tr is cerium.

**13.** A process according to any one of claims 1 to 10 wherein there is used a catalyst of the formula (I) wherein Tr is lanthanum.

**14.** A process according to any one of claims 1 to 10 wherein there is used a catalyst of the formula (I) wherein Tr is praeseodymium.

**15.** A process according to any one of claims 1 to 10 wherein there is used a catalyst of the formula (I) wherein Tr is neodymium.

**16.** A process according to any one of the preceding claims wherein the rare earth oxyhydride is prepared by a process comprising the steps as follows:-

in a first step subjecting an IREC of the formula:

$Tr_{1-g}G_gNi_{5-p}M_p$ (XI)

in a closed reactor, preferably an autoclave, to a plurality of hydriding/dehydriding cycles thereby to form a hydride, the hydriding being performed under a hydrogen pressure of from 20 to 200, preferably from 50 to 100 bar, and at a temperature of from 20 to 450°C, preferably about 350°C, and the dehydriding being performed in the same closed reactor at a temperature in the range from 20 to 450°C,

in a second step oxidising the hydrided product obtained in the first step, and

in a third step chemically reducing the product obtained in the second step.

**17.** A process according to any one of claims 1 to 15 wherein the rare earth oxyhydride is prepared by a process comprising the steps as follows:-

in a first step forming a solution comprising soluble salts of nickel and the other metallic element(s) thermally decomposable to the oxides thereof in such proportions as to satisfy the stoichiometric relationship of formula (I), and thereafter precipitating a mixture of the thermally decomposable salts of nickel and the other element(s),

in a second step partially or wholly thermally decomposing the mixture of heat decomposable salts obtained in the first step, and

in a third step reducing the product obtained in the second step in the present of hydrogen.

**18.** A process according to any one of claims 1 to 15 wherein the rare earth oxyhydride is prepared by the method of claim 17 in which precipitation of the mixture of the thermally decomposable salts of nickel and the other element(s) is accomplished by adding the solution of salts portionwise to an excess of the base under conditions of efficient mixing, thereby to effect precipitation at constant pH.

**19.** A process for the production of a rare earth oxhydride of formula (I) which process comprises the steps as follows:-

in a first step forming a solution comprising soluble salts of nickel and the other metallic element(s) thermally decomposable to the oxides thereof in such proportions as to satisfy the stoichiometric relationship of formula (I), and thereafter precipitating a mixture of the thermally decomposable salts of nickel and the other element(s) by adding the solution of the salts portionwise to an excess of a basic solution under conditions of efficient mixing, thereby to effect precipitation at constant pH,

in a second step partially or wholly thermally decomposing the mixture of heat decomposable salts obtained in the first step, and

in a third step reducing the product obtained in the second step in the presence of hydrogen.

## Revendications

**1.** Procédé d'hydrogénation d'un composé organique insaturé par mise en contact du composé avec un catalyseur d'hydrogénation solide dans des conditions d'hydrogénation, caractérisé en ce que le catalyseur d'hydrogénation solide comprend un oxyhydrure de terres rares de la formule :

$Tr_{1-q}G_qNi_{5-p}M_pO_yH_x$

où Tr est un élément des terres rares,

G est Th et/ou Y,

M est un métal choisi dans la classe formée par Cu, Mn, Al, Fe, Cr, Co, Rh, Ru, Re, Pt, Pd, Ir, Os, Mo, V, Ti, W et les mélanges de deux ou plusieurs d'entre eux,

q est zéro ou un nombre inférieur ou égal à 1,

p est zéro ou un nombre inférieur ou égal à 5,

y est un nombre entier ou un nombre décimal de l'intervalle de 0,4 à 6,5, et

x est zéro ou un nombre entier ou un nombre décimal égal ou inférieur à 15,

avec la restriction que le procédé est autre que l'hydrogénation de l'isoprène en présence d'un oxyhydrure de la stoechiométrie $MmNi_5O_{1.7-w}(NO_3)_{2w}H_8$ et d'une surface spécifique d'à peu près 20 $m^2$ par g.

**2.** Procédé suivant la revendication 1, dans lequel le composé organique insaturé est un composé oléfiniquement insaturé.

**3.** Procédé suivant la revendication 1 ou 2, dans lequel le composé insaturé est choisi parmi les oléfines, les hydrocarbures aromatiques monocycliques, les hydrocarbures aromatiques polycycliques, les hydrocarbures aromatiques hétérocycliques contenant de l'azote dans le cycle aromatique, les hydrocarbures aromatiques hétérocycliques contenant du soufre dans le cycle aromatique, et un mélange de deux ou plusieurs d'entre eux.

**4.** Procédé suivant la revendication 1, dans lequel le composé organique insaturé est l'isoprène.

**5.** Procédé suivant la revendication 1, dans lequel le composé organique insaturé est le naphtalène.

**6.** Procédé suivant la revendication 1, dans lequel le composé organique insaturé est la pyridine.

**7.** Procédé suivant la revendication 1, dans lequel le composé organique insaturé est le thiophène.

**8.** Procédé suivant la revendication 1, dans lequel le procédé d'hydrogénation est un hydrotraitement d'une alimentation issue d'une huile minérale.

**9.** Procédé suivant la revendication 1, dans lequel l'hydrotraitement est un hydrocraquage, une hydrodésulfuration, une hydrodésazotation, une hydroisomérisation ou une hydrogénation.

**10.** Procédé suivant l'une quelconque des revendications précédentes, dans lequel le procédé est exécuté en présence d'un supplément d'hydrogène.

**11.** Procédé suivant l'une quelconque des revendications précédentes, dans lequel il est fait usage d'un

catalyseur de formule (I), où Tr est le mischmétal.

12. Procédé suivant l'une quelconque des revendications 1 à 10, dans lequel il est fait usage d'un catalyseur de formule (I), où Tr est le cérium.

13. Procédé suivant l'une quelconque des revendications 1 à 10, dans lequel il est fait usage d'un catalyseur de formule (I), où Tr est le lanthane.

14. Procédé suivant l'une quelconque des revendications 1 à 10, dans lequel il est fait usage d'un catalyseur de formule (I), où Tr est le praséodyme.

15. Procédé suivant l'une quelconque des revendications 1 à 10, dans lequel il est fait usage d'un catalyseur de formule (I), où Tr est le néodyme.

16. Procédé suivant l'une quelconque des revendications précédentes, dans lequel l'oxyhydrure de terres rares est préparé par un procédé comprenant les stades suivants :
à un premier stade, l'exécution sur un CITR de formule :

$Tr_{1-g}G_gNi_{5-p}M_p$ (XI)

dans un réacteur clos, de préférence un autoclave, d'une pluralité de cycles d'hydruration/déshydruration pour former un hydrure, l'hydruration étant exécutée sous une pression d'hydrogène de 20 à 200 et de préférence de 50 à 100 bars et à une température de 20 à 450°C, de préférence d'environ 350°C, et la déshydruration étant exécutée dans le même réacteur clos à une température de l'intervalle de 20 à 450°C,
à un deuxième stade, l'oxydation du produit hydruré obtenu au premier stade, et
à un troisième stade, la réduction chimique du produit obtenu au deuxième stade.

17. Procédé suivant l'une quelconque des revendications 1 à 15, dans lequel l'oxyhydrure de terres rares est préparé par un procédé comprenant les stades suivants :
à un premier stade, la formation d'une solution comprenant des sels solubles de nickel et du ou des autres éléments métalliques thermiquement décomposables en leurs oxydes en proportions de nature à satisfaire à la relation stoechiométrique de la formule (I), et ensuite la précipitation d'un mélange des sels thermiquement décomposables de nickel et du ou des autres éléments,
à un deuxième stade, la décomposition thermique partielle ou totale du mélange des sels thermiquement décomposables obtenus au premier stade, et
à un troisième stade, la réduction du produit obtenu au deuxième stade, en présence d'hydrogène.

18. Procédé suivant l'une quelconque des revendications 1 à 15, dans lequel l'oxyhydrure de terres rares est préparé par le procédé suivant la revendication 17, dans lequel la précipitation du mélange des sels thermiquement décomposables de nickel et du ou des autres éléments est effectuée en ajoutant la solution des sels peu à peu à un excès de la base dans des conditions de mélange efficace, de manière à opérer la précipitation à pH constant.

19. Procédé de production d'un oxyhydrure de terres rares de formule (I), lequel procédé comprend les stades suivants :
à un premier stade, la formation d'une solution comprenant des sels solubles de nickel et du ou des autres éléments métalliques thermiquement décomposables en leurs oxydes en proportions de nature à satisfaire à la relation stoechiométrique de la formule (I) et ensuite la précipitation d'un mélange des sels thermiquement décomposables de nickel et du ou des autres éléments métalliques par addition de la solution des sels peu à peu à un excès de solution basique dans des conditions de mélange efficace, de manière à opérer la précipitation à pH constant,
à un deuxième stade, la décomposition thermique partielle ou totale du mélange de sels thermiquement décomposables obtenus au premier stade, et
à un troisième stade, la réduction du produit obtenu au deuxième stade, en présence d'hydrogène.

**Patentansprüche**

1. Verfahren zur Hydrierung einer ungesättigten organischen Verbindung, durch In-Kontakt-Bringen der Verbindung mit einem festen Hydrierungskatalysator unter Hydrierungsbedingungen, dadurch gekennzeichnet, daß der feste Hydrierungskatalysator ein Seltenenerd-Oxyhydrid mit der Formel: $Tr_{1-q}G_qNi_{5-p}M_pO_yH_x$ umfaßt, worin
Tr ein Seltenerdmetall ist, G entweder Th und/oder Y ist,
M ein Metall aus der Gruppe bestehend aus Cu, Mn, Al, Fe, Cr, Co, Rh, Ru, Re, Pt, Pd, Ir, Os, Mo, V, Ti, W und Mischungen aus 2 oder mehreren davon ist,
q Null oder eine Zahl kleiner oder gleich 1 ist,
p Null oder eine Zahl kleiner oder gleich 5 ist,
y eine ganze Zahl oder Dezimalzahl in dem Bereich von 0,4 bis 6,5 ist und
x Null oder eine ganze Zahl oder Dezimalzahl gleich oder kleiner als 15 ist und mit der Maßgabe, daß das Verfahren anders ist, als die Hydrierung von Isopren in Anwesenheit eines Oxyhydrids mit der Stöchiometrie $MmNi_5O_{1.7-w}(NO_3)_{2w}H_8$ und einer spezifischen Oberfläche von ungefähr 20 $m^2/g$.

2. Verfahren nach Anspruch 1, worin die ungesättigte organische Vebindung eine olefinisch ungesättigte Verbindung ist.

3. Verfahren nach Anspruch 1 oder 2, worin die ungesättigte Verbindung ausgewählt wird aus Olefinen, monocyklischen aromatischen Kohlenwasserstoffen, polycyklischen aromatischen Kohlenwasserstoffen, Stickstoff im aromatischen Ring enthaltenden heterocyclischen aromatischen Kohlenwasserstoffen und heterocyklischen aromatischen Kohlenwasserstoffen, die Schwefel im aromatischen Ring enthalten oder einem Gemisch von zwei oder mehreren davon.

4. Verfahren nach Anspruch 1, worin die ungesättigte organische Verbindung Isopren ist.

5. Verfahren nach Anspruch 1, worin die ungesättigte organische Verbindung Naphthalin ist.

6. Verfahren nach Anspruch 1, worin die ungesättigte organische Verbindung Pyridin ist.

7. Verfahren nach Anspruch 1, worin die ungesattigte organische Verbindung Thiophen ist.

8. Verfahren nach Anspruch 1, worin das Hydrierungsverfahren eine Hydro-Behandlung eines von Erdöl abgeleiteten Einsatzproduktes ist.

9. Verfahren nach Anspruch 8, worin die Hydro-Behandlung entweder eine Hydrocrackreaktion, eine Hydrodesulfurisierung, eine Hydrodenitrifizierung, eine Hydroisomerisierung oder Hydrierung ist.

10. Verfahren nach einem der vorhergehenden Ansprüche, worin das Verfahren in Anwesenheit von zusätzlichem Wasserstoff durchgeführt wird.

11. Verfahren nach einem der vorhergehenden Ansprüche, worin ein Katalysator mit der Formel (I), wobei Tr ein Mischmetall ist, verwendet wird.

12. Verfahren nach einem der Ansprüche 1 bis 10, worin ein Katalysator mit der Formel (I) , wobei Tr Cer ist, verwendet wird.

13. Verfahren nach einem der Ansprüche 1 bis 10, worin ein Katalysator mit der Formel (I), worin Tr Lanthan ist, verwendet wird.

14. Verfahren nach einem der Ansprüche 1 bis 10, worin ein Katalysator mit der Formel (I), worin Tr Praseodym ist, verwendet wird.

15. Verfahren nach einem der Ansprüche 1 bis 10, worin ein Katalysator mit der Formel (I), worin Tr Neodym ist, verwendet wird.

16. Verfahren nach einem der vorhergehenden Ansprüche, worin das Seltenerd-Oxyhydrid durch ein Verfahren, das die folgenden Schritte umfaßt, hergestellt wird:
in einem erstem Schritt wird ein IREC der Formel

$Tr_{1-g}G_gNi_{5-p}M_p$ (XI)

in einem geschlossenen Reaktor, vorzugsweise einem Autoklaven, einer Vielzahl von Hydrierungs/Dehydrierungszyklen ausgesetzt, um dadurch ein Hydrid zu bilden, wobei die Hydrierung bei einem Wasserstoffdruck von 20 bis 200, vorzugsweise von 50 bis 100 bar, ausgeführt wird, und bei einer Temperatur von 20 bis 450 °C, vorzugsweise bei ungefähr 350 °C, und wobei die Dehydrierung in demselben geschlossen Reaktor bei einer Temperatur im Bereich von 20 bis 450 °C durchgeführt wird,
in einem zweiten Schritt wird das aus dem ersten Schritt erhaltene hydrierte Produkt, oxidiert und
in einem dritten Schritt wird das in dem zweiten Schritt erhaltene Produkt chemisch reduziert.

**17.** Verfahren nach einem der Ansprüche 1 bis 15, worin das Seltenerd-Oxyhydrid durch ein Verfahren, das die folgenden Schritte umfaßt, hergestellt wird:
in einem ersten Schritt wird eine Lösung, umfaßend lösliche Salze von Nickel und des(den) anderen metallischen Elements (Elementen) gebildet, welche thermisch zu ihren Oxiden zersetzbar sind, in einem solchen Verhältnis, daß das stöchiometrische Verhältnis der Formel (I) erfüllt wird, und danach wird ein Gemisch der thermisch zersetzbaren Salze von Nickel und des(den) anderen Elements-(Elementen) ausgefällt,
in einem zweiten Schritt wird das im ersten Schritt erhaltene Gemisch thermisch zersetzbarer Salze, teilweise oder ganz thermisch zersetzt, und
in einem dritten Schritt wird das im zweiten Schritt erhaltene Produkt in Anwesenheit von Wasserstoff reduziert.

**18.** Verfahren nach einem der Ansprüche 1 bis 15, worin das Seltenerd-Oxyhydrid nach dem Verfahren von Anspruch 17 hergestellt wird, bei dem das Ausfällen des Gemischs, der thermisch zersetzbaren Salze von Nickel und dem(den) anderen Element (Elementen) durch portionsweises Hinzufügen der Salzlösung zu einem Basenüberschuß bei genügendem Vermischen bewirkt wird, wodurch eine Ausfällung bei konstantem pH-Wert bewirkt wird.

**19.** Verfahren zur Herstellung eines Seltenerd-Oxyhydrids der Formel (I), wobei das Verfahren die folgenden Schritte umfaßt:
in einem ersten Schritt wird eine Lösung, die lösliche Salze von Nickel und dem(den) anderen metallischen Element(Elementen) umfaßt, welche thermisch in ihre Oxide zersetzbar sind, in solchen Verhältnissen, daß das stöchiometrische Verhältnis der Formel (I) erfüllt wird, hergestellt, und danach wird ein Gemisch der thermisch zersetzbaren Salze von Nickel und dem(den) anderen Element-(Elementen) ausgefällt durch portionsweises Hinzufügen der Salzlösung zu einem Überschuß einer basischen Lösung unter wirksamen Rührbedingungen, wobei eine Ausfällung bei konstantem pH-Wert erfolgt,
in einem zweiten Schritt wird das aus dem ersten Schritt erhaltene Gemisch der thermisch zersetzbaren Salze teilweise oder ganz thermisch zersetzt, und
in einem dritten Schritt wird das im zweiten Schritt erhaltene Produkt in Anwesenheit von Wasserstoff reduziert.

FIG. 1

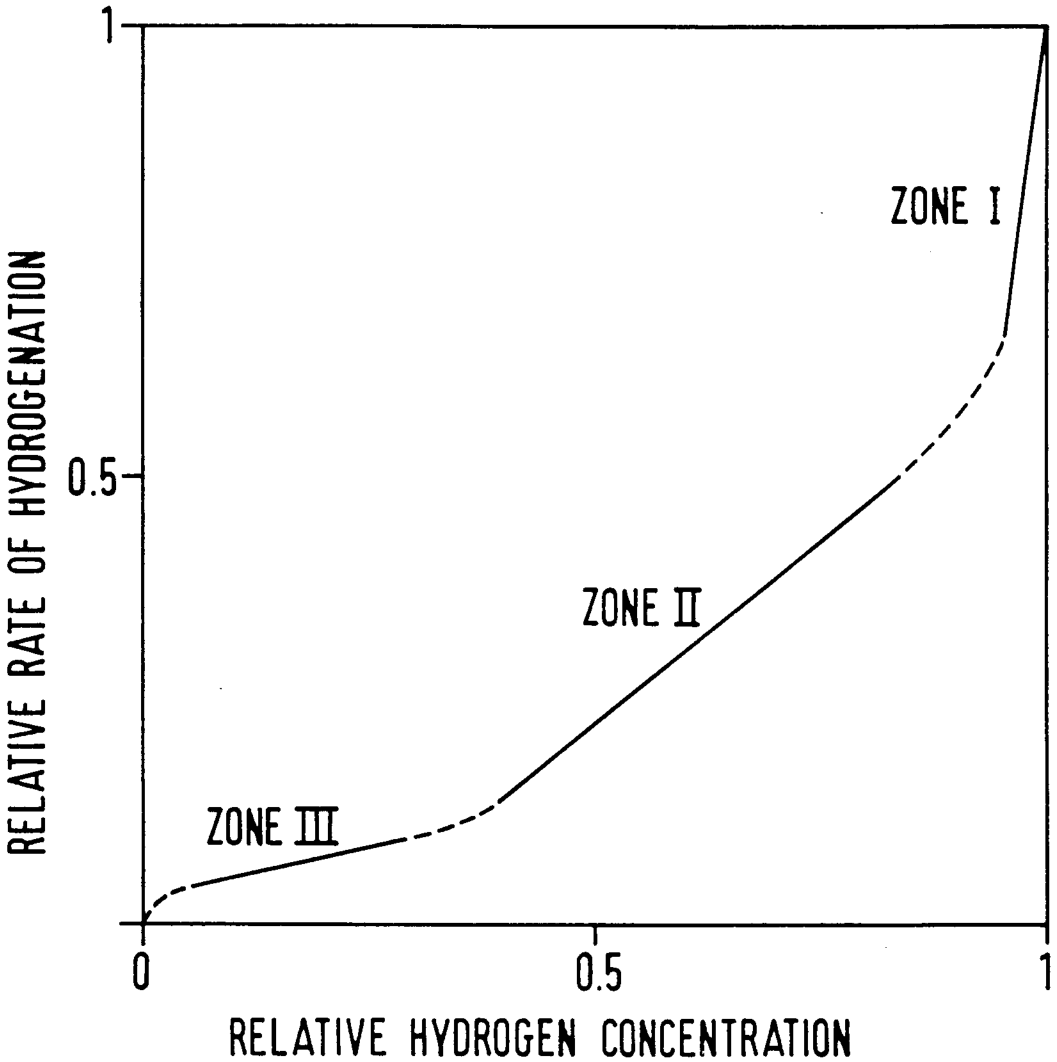
1
0.5
0
0.5
1
ZONE I
ZONE II
ZONE III
RELATIVE RATE OF HYDROGENATION
RELATIVE HYDROGEN CONCENTRATION

FIG. 2

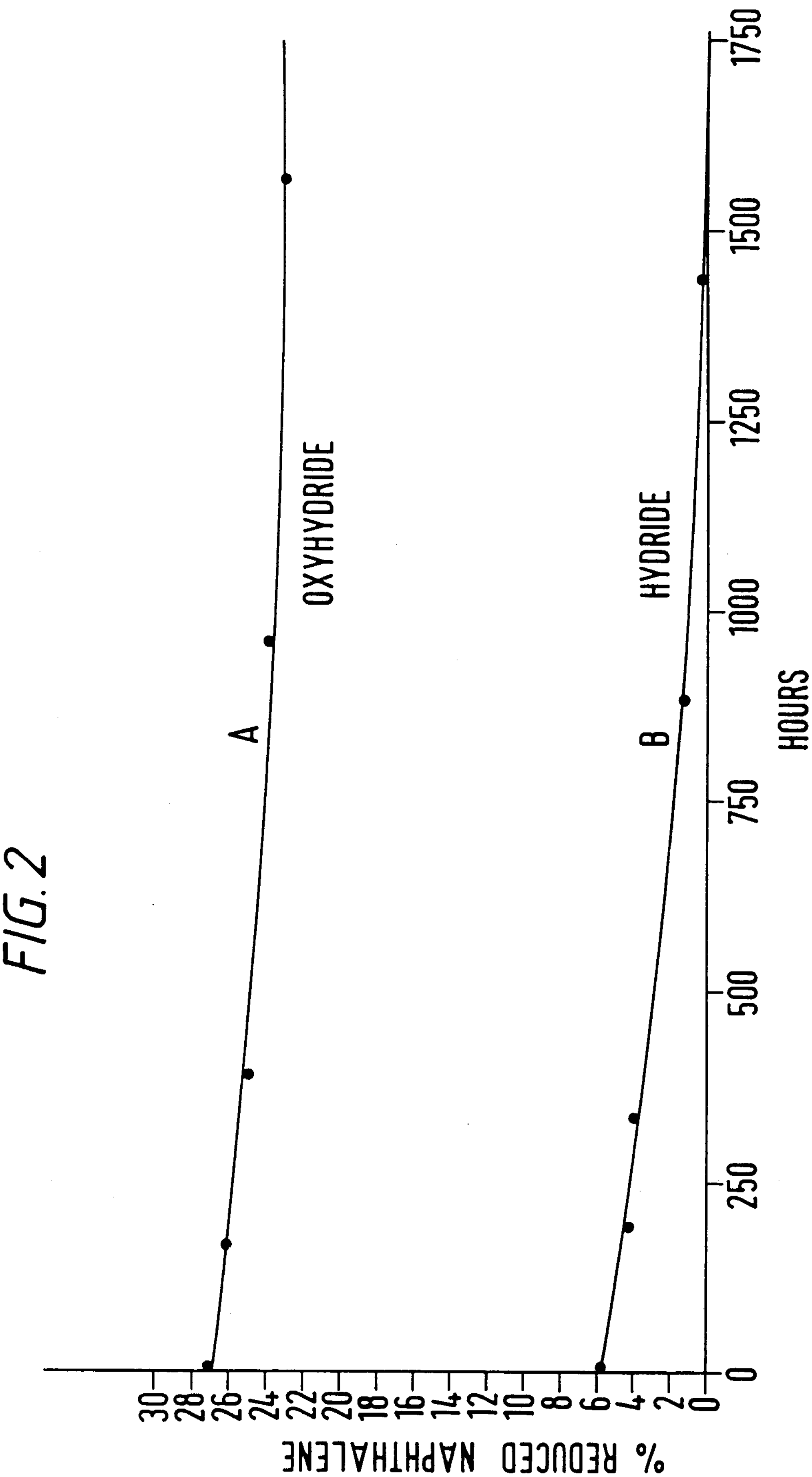
A
OXYHYDRIDE
B
HYDRIDE
% REDUCED NAPHTHALENE
30
28
26
24
22
20
18
16
14
12
10
8
6
4
2
0
0
250
500
750
1000
1250
1500
1750
HOURS

FIG. 3

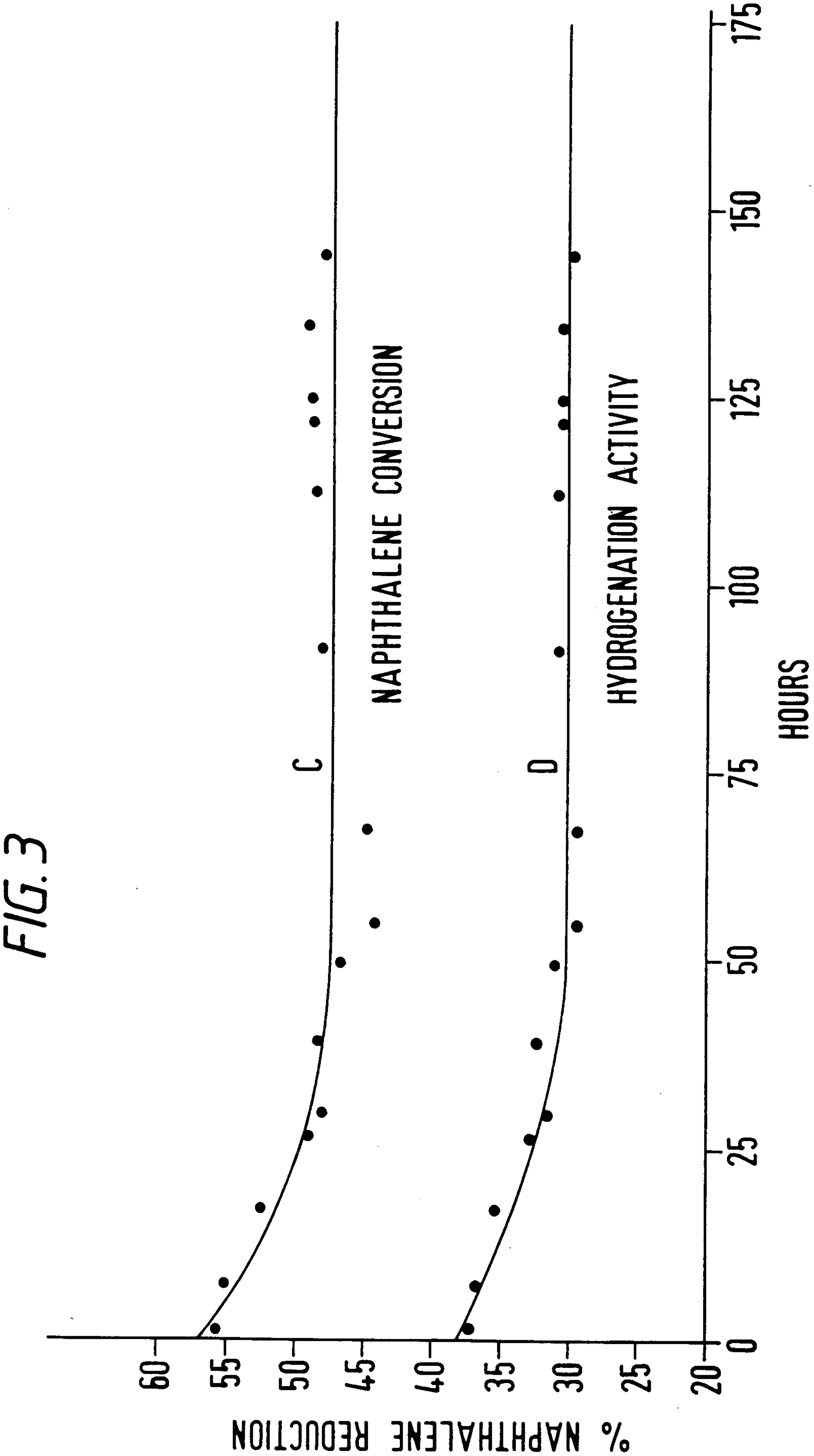
% NAPHTHALENE REDUCTION
60
55
50
45
40
35
30
25
20
0
25
50
75
100
125
150
175
HOURS
C
NAPHTHALENE CONVERSION
D
HYDROGENATION ACTIVITY

FIG. 4

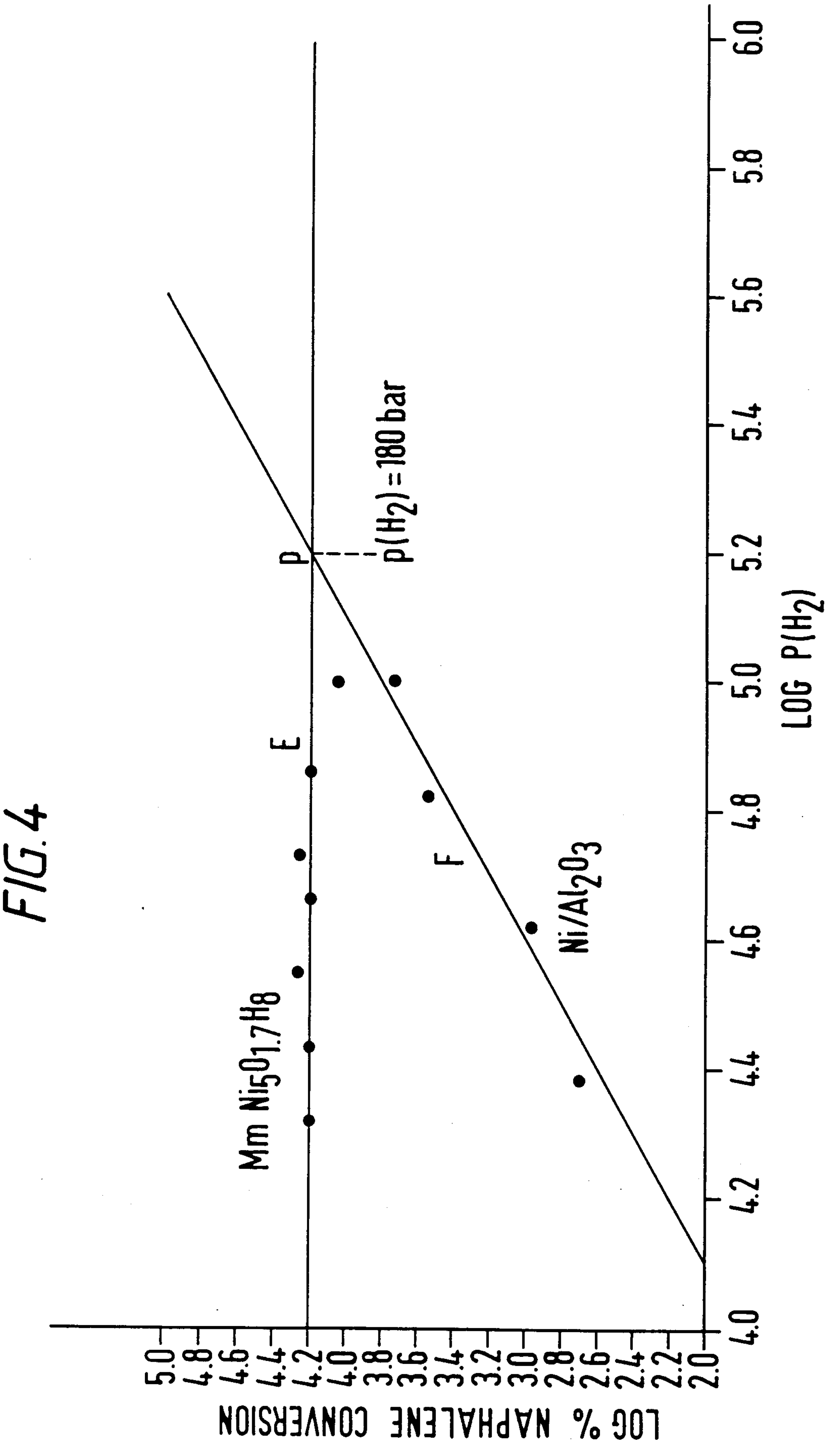
LOG % NAPHALENE CONVERSION
5.0
4.8
4.6
4.4
4.2
4.0
3.8
3.6
3.4
3.2
3.0
2.8
2.6
2.4
2.2
2.0
LOG P(H2)
4.0
4.2
4.4
4.6
4.8
5.0
5.2
5.4
5.6
5.8
6.0
Mm Ni5O1.7H8
E
P
F
Ni/Al2O3
p(H2) = 180 bar